(19) Europäisches Patentamt
European Patent Office
Office européen des brevets

(11) **EP 4 065 071 B1**

(12) **EUROPEAN PATENT SPECIFICATION**

(45) Date of publication and mention
of the grant of the patent:
**17.09.2025 Bulletin 2025/38**

(21) Application number: **20800072.9**

(22) Date of filing: **28.10.2020**

(51) International Patent Classification (IPC):
*A61K 8/37* (2006.01)        *A61K 8/34* (2006.01)
*A61Q 5/12* (2006.01)        *A61K 8/33* (2006.01)
*A61K 8/41* (2006.01)

(52) Cooperative Patent Classification (CPC):
**A61Q 5/12; A61K 8/33; A61K 8/342; A61K 8/37;
A61K 8/416**

(86) International application number:
**PCT/EP2020/080273**

(87) International publication number:
**WO 2021/104786 (03.06.2021 Gazette 2021/22)**

(54) **A CONDITIONING COMPOSITION**

PFLEGENDE ZUSAMMENSETZUNG

COMPOSITION DE CONDITIONNEMENT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.11.2019 PCT/CN2019/121114**

(43) Date of publication of application:
**05.10.2022 Bulletin 2022/40**

(73) Proprietor: **Beiersdorf AG
22529 Hamburg (DE)**

(72) Inventors:
• **FENG, Ji
Wuhan, 420070 (CN)**

• **ZIA, Caroline
22767 Hamburg (DE)**
• **JIA, Zebao
Wuhan, 430070 (CN)**

(74) Representative: **Beiersdorf AG
Patentabteilung
Beiersdorfstraße 1 - 9
22529 Hamburg (DE)**

(56) References cited:
**WO-A1-2015/066777        WO-A1-2016/090150**

• **DATABASE WPI Week 201022, Derwent World
Patents Index; AN 2009-K33099, XP002801704**

## Description

### Technical field

The present invention relates to a cosmetic composition, especially a conditioning composition. The composition has a light texture and at the same time provides a caring effect to hair.

### Background art

The entire human body, with the exception of the lips, the palms of the hands and the soles of the feet, is covered with hair, albeit for a large part barely visible. Because of many nerve endings at the hair root, hair reacts sensitively to external influences such as wind or touch and is therefore a part of the sense of touch that should not be underestimated. However, nowadays, the most important function of human head hair lies in helping to create the appearance of the person in a characteristic manner. Similarly to the skin, it fulfils a social function because it contributes considerably to interpersonal relations and to the self-esteem of an individual via its outward appearance.

Hair consists of hair shaft and hair root. Hair shaft protrudes freely from the skin, and is a keratinized (dead) section of the hair. Hair shaft represents the actual visible part of the hair, which is continually renewed. Hair root which sticks in the skin is the living part of the hair. Hair shaft consists of three layers: the central part, which is called hair marrow (medulla), regressed in humans, often completely missing; then the marrow, also called cortex; and cuticle as the outermost layer, comprising up to ten horny layers.

Human hair in its freshly grown condition is virtually impossible to improve. The part of the hair in the vicinity of the scalp accordingly has a virtually closed horny layer. In particular, the horny layer, being the external sheath of the hair, and also the inner region below the cuticle are subjected to particular stress by environmental influences.

To keep the hair in a condition resembling freshly grown hair, many consumers apply products with caring and nourishing properties to the hair. Today, the daily routine comprises a step of washing hair and frequently a step of drying the hair using blow-dryer or other drying means, where the hair is exposed to relatively high temperatures. In the long term this step may result in dry hair, which is often only hardly manageable.

Moreover, the hair may be colored, in a permanent or non-permanent way and/or the hair may be permed or uncurled. All these procedures involve the application of sometimes relatively aggressive chemical compounds and together with high temperatures.

These treatments stress the hair resulting in dry, dull, and hardly manageable hair. To improve these undesirable conditions of the hair, many products are on the market. A very famous and often used product is a conditioner, which may be a separate product or included in a shampoo as a 2-in-1 preparation.

Conditioning compositions are known in prior art; only a few will be cited.

WO 2017/036134 A1 describes the effect of lotus flower oil in a hair conditioning composition.

WO 2018/018487 A1 describes the effect of rose hip oil in a hair conditioning composition.

WO 2018/227515 A1 describes the effect of a Cymbidium Grandiflorum Flower Extract in a hair conditioning composition.

WO 2018/095152 A1 discloses a transparent conditioning composition.

WO 2016/090150 A1, WO 2015/066777 A1 and the disclosure Database WPI, week 201022, Thomson Scientific, London, GB; AN 2009-K33099 & KR 2009 0054113 A (HO G B) 29 May 2009 describe silicone-free compositions suitable as conditioning rinse-off.

The main purpose of a conditioner is to provide care for the hair, helping to improve the condition of hair. For this issue, components having a caring effect are included in the conditioner. Such components comprise silicone compounds, natural oils, fatty alcohols, cationic polymers and surfactants, and many more. In many cases it could be shown that the resulting conditioning composition has a caring effect to hair, but has drawbacks, too. Frequently, said compositions have a compact texture. Said compositions may be difficult to be released from the packaging device and also the distribution on hair is impaired. Moreover, the hair may be weighed down and have little volume; sometimes hair even looks greasy. Consumers do not like products having the above mentioned negative properties.

Therefore, a conditioner should be made available having a light texture and at the same time providing a caring effect to the hair, which is at least comparable to the one known conditioners show.

### Summary of the invention

Surprisingly, the above mentioned problem could be solved by a conditioning composition containing

- at least one emollient chosen from at least one ester oil, one semi synthetic triglyceride, one alkane having from 8 to 20 carbon atoms, and/or one poly-C2-C16-olefin, especially one hydrogenated poly-C2-C16-olefin,

- at least one emulsifier, preferably at least one O/W emulsifier and/or at least one cationic surfactant,
- a combination of fatty alcohols, wherein one or more fatty alcohol(s) having 18 or more carbon atoms is/are contained in a total amount of less than 2.0 % by weight and one or more fatty alcohol(s) having 16 or less carbon atoms is/are contained in a total amount of less than 6.0 % by weight, respectively each one in relation to the total weight of the composition, and

wherein the composition is free from silicones,
wherein the at least one emulsifier is contained in a total amount of 0.05 to 3.0 % by weight, in relation to the total weight of the composition, and
wherein the at least one O/W emulsifier is chosen from glyceryl stearate citrate, glyceryl stearate SE, polyglyceryl-3 distearate in combination with glyceryl stearate citrate, and/or polyglyceryl-3 methyl glucose distearate, and
wherein water is contained in an amount of at least 75 % by weight, preferably 85 to 98 % by weight water, more preferably 88 to 95 % by weight water, in relation to the total weight of the composition.

The conditioning composition is an aqueous composition containing at least 75 % by weight water, preferably 85 to 98 % by weight water, more preferably 88 to 95 % by weight water.

The conditioning composition is preferably provided in form of an emulsion, preferably in form of an O/W emulsion.

In general, it is understood that emulsions are heterogeneous systems, which consist of two liquids which are immiscible or have only limited miscibility with one another and which are usually referred to as phases. In an emulsion, one of the two liquids is dispersed in the form of very fine droplets in the other liquid. The liquids (pure or as solutions) are present in an emulsion in a more or less fine distribution, which is generally of only limited stability.

If the two liquids are water and oil and oil droplets are present in finely distributed form in water, then this is an oil-in-water emulsion (O/W emulsion, e.g. milk). The basic character of an O/W emulsion, exemplified by electrical conductivity, sensory properties, ability of the continuous phase to be stained, is determined by the water. In the case of a water-in-oil emulsion (W/O emulsion, e.g. butter), the basic character is determined by the oil.

To stabilize an emulsion, one or more emulsifier(s) is/are contained in general.

Emulsifiers help to combine two immiscible liquids (for example oil in water) in a way to result in a stable preparation, an emulsion. For that reason emulsifiers have to have an amphiphilic character, the hydrophobic part interacts with the oily or lipid phase and the hydrophilic part interacts with the aqueous phase. By stirring or homogenizing the generated droplets are dispersed in the respective environment, namely aqueous droplets in a lipid environment or lipid droplets in an aqueous environment. Primarily, emulsifiers do not have a detersive, surfactant character. Emulsifiers reduce the interfacial tension between the two phases and, besides reducing the interfacial work, also achieve a stabilization of the emulsion formed. They stabilize the formed emulsion by means of interfacial films, as well as by forming steric or electrical barriers, as a result of which the merging (coalescence) of the emulsified particles is prevented.

HLB values are suitable to characterize emulsifiers, said values specify the hydrophilicity of a given emulsifier. The HLB value may be determined by the following formula:

$$HLB = 20 \times (1 - M_{lipophile}/M),$$

where $M_{lipophile}$ represents the molar mass of the lipophilic fraction of a given emulsifier and M represents the molar mass of the total emulsifier.

In general, emulsifiers with an HLB value up to about 8 are considered to be W/O-emulsifiers. By contrast, O/W-emulsifiers have HLB values of greater than 8 to 15. Substances with HLB values greater than 15 are often referred to as solubilizers.

Suitable emulsifiers may be chosen from:
Ceteareth-12, ceteareth-20, ceteareth-25, ceteareth-3, ceteth-10, ceteth-2, ceteth-20, cetyl phosphate, cetyl stearyl alcohol in combination with PEG-20 stearate, glyceryl stearate citrate, glyceryl stearate in combination with PEG-100 stearate, glyceryl stearate in combination with PEG-30 stearate, glyceryl stearate SE, isosteareth-10, isosteareth-20, isostearyl diglyc-eryl succinate, laureth-23, laureth-4 phosphate, laureth-4, methyl glucose sesquistearate, PEG-100 stearate, PEG-20 glyceryl stearate, PEG-20 stearate, PEG-20 methyl glucose sesquistearate, PEG-30 stearate, PEG-40 stearate, PEG-45/dodecyl glycol copolymer, PEG-6 caprylic/capric glyceride, polyglyceryl-10 stearate, polyglyceryl-2 laurate, polyglyceryl-2 PEG-4 stearate, polyglyceryl-2 sesquiisostearate, polyglyceryl-3 distearate, polyglyceryl-3 distearate in combination with glyceryl stearate citrate, polyglyceryl-3 methyl glucose distearate, polysorbate 60, potassium cetyl phosphate, sodium cetyl stearyl sulfate, sodium stearoyl glutamate, steareth-10, steareth-2, steareth-20, steareth-21, stearic acid, sucrose polystearate + hydrogenated polyisobutene, triceteareth-4 phosphate, trilaureth-4 phosphate.

Glyceryl stearate citrate, glyceryl stearate SE, polyglyceryl-3 distearate in combination with glyceryl stearate citrate, and/or polyglyceryl-3 methyl glucose distearate, preferably polyglyceryl-3 methyl glucose distearate are contained in the composition of the present invention.

The at least one emulsifier is contained in a total amount of 0.05 to 3.0 % by weight, preferably 0.1 to 2.0 % by weight, in relation to the total weight of the composition. If the emulsifier is a single component, the values refer to active content. If the emulsifier is a mixture of two or more components the values refer to the amount of the mixture.

According to the invention the at least one emollient may be chosen from esters of saturated and/or unsaturated, branched and/or unbranched alkane carboxylic acids with a chain length of from 3 to 20 carbon atoms and saturated and/or unsaturated, branched and/or unbranched alcohols with a chain length of from 1 to 20 carbon atoms, and esters of aromatic carboxylic acids and saturated and/or unsaturated, branched and/or unbranched alcohols with a chain length of from 3 to 20 carbon atoms. Such components may be termed ester oils. Examples for suitable ester oils are octyl palmitate, octyl cocoate, octyl isostearate, octyldodecyl myristate, cetearyl isononanoate, isopropyl myristate, isopropyl palmitate, isopropyl stearate, isopropyl oleate, n-butyl stearate, n-hexyl laurate, n-decyl oleate, isooctyl stearate, isononyl stearate, isononyl isononanoate, 2-ethylhexyl palmitate, 2-ethylhexyl laurate, 2-ethylhexyl stearate, 2-hexyldecyl stearate, 2-octyldodecyl palmitate, stearyl heptanoate, oleyl oleate, oleyl erucate, erucyl oleate, erucyl erucate, tridecyl stearate, tridecyl trimellitate, isoamyl laurate.

It is preferred, if the at least one ester oil is selected from esters of alcohols with a small number of carbon atoms, i.e. having 2 to 8 carbon atoms, for instance such as isopropanol, propylenglycol, neopentyl glycol and isopentyl alcohol and fatty acids, preferably isopentyl alcohol and isopropanol. More preferably the at least one ester oil is chosen from isopropyl myristate and/or isoamyl laurate. Isopropyl myristate (IPM) may be purchased from BASF and isoamyl laurate may be purchased as Mackaderm Lia from Solvay.

If the at least one ester oil is contained in the composition of the present invention, the at least one ester oil is contained in a total amount of 0.1 to 5.0 % by weight, preferably 0.4 to 3.0 % by weight, in relation to the total weight of the composition.

The at least one emollient may be selected from at least one poly-C2-C16-olefin, in particular chosen from polybutene, polyisobutene and polydecene. Preferably, the at least one poly-C2-C16-olefin is contained in hydrogenated form, more preferably hydrogenated polyisobutene and/or hydrogenated Polydecene are contained. Hydrogenated polydecene may be purchased from The Innovation Companyas Alphaflow® 40 or from Nisshin Oillio Group as OMCORT HP-100 or from Exxon Mobil Corporation as SPECTRASYN 4 or from Chevron Phillips Chemicals as Dekanex 2006 FG.

If at least one emollient in form of at least one poly-C2-C16-olefin is contained in the composition of the present invention, the at least one poly-C2-C16-olefin is contained in a total amount of 0.05 to 6.0 % by weight, preferably 0.1 to 4.0 % by weight, relative to the total weight of the composition.

The at least one emollient may be selected from triglycerides. Triglycerides are ester of one molecule of glycerine and three saturated and/or unsaturated, branched and/or unbranched carboxylic acids having a chain length of 8 to 24, especially 12 to 18 carbon atoms. The triglycerides may be classified as synthetic, semi synthetic and natural ones.

According to the invention, at least one semi synthetic triglyceride may be contained. An example of a preferred semi synthetic triglyceride is caprylic/capric triglyceride, which may be produced starting from palm kernel oil or coconut oil. The respective oils are saponified resulting in glycerine and the respective fatty acids. The fatty acids not being capric acid and caprylic acid are discarded and only capric acid and caprylic acid are again esterified with glycerine.

If at least one emollient in form of at least one semi synthetic triglyceride is contained in the composition of the present invention, the at least one semi synthetic triglyceride is contained in a total amount of 0.05 to 6.0 % by weight, preferably 0.1 to 4.0 % by weight, relative to the total weight of the composition.

The at least one emollient may be selected from at least one alkane having from 8 to 20 carbon atoms, especially mixtures thereof. A suitable example is the mixture of undecane/tridecane, which is a natural-based emollient, being prepared from 100% renewable components. Undecane/tridecane is described as a possible substitute for cyclomethicones. Undecane/tridecane may be purchased from BASF as Cetiol Ultimate.

If the at least one emollient in form of at least one alkane having from 8 to 20 carbon atoms is contained in the composition of the present invention, the at least one alkane having from 8 to 20 carbon atom is contained in a total amount of 0.05 to 6.0% by weight, preferably 0.1 to 4.0 % by weight, relative to the total weight of the composition.

In the composition of the present invention a combination of fatty alcohols is contained. Said combination is characterized in comprising one or more fatty alcohol(s) having 18 or more carbon atoms contained in a total amount of less than 2.0 % by weight and one or more fatty alcohol(s) having 16 or less carbon atoms contained in a total amount of less than 6.0 % by weight, respectively each one in relation to the total weight of the composition.

Fatty alcohols are straight, long-chained molecules with one hydroxyl group at the first carbon atom. The alkyl residue has 6 to 22 carbon atoms and may have one or multiple double bonds.

It is preferred if stearyl alcohol is one of the alcohols from the group of fatty alcohols having 18 or more carbon atoms.

It is likewise preferred, if myristyl alcohol and/or cetyl alcohol is/are part of the group of fatty alcohols having 16 or less carbon atoms.

It is more preferred, if stearyl alcohol, myristyl alcohol and cetyl alcohol are contained in the composition of the present invention.

It is most preferred, if in the composition of the present invention the combination of fatty alcohols consists of stearyl alcohol, myristyl alcohol and cetyl alcohol.

Preferably, in the composition according to the present invention one or more fatty alcohol(s) having 18 or more carbon atoms is/are contained in a total amount of less than 1.8 % by weight, preferably less than 1.5 % by weight, in relation to the total weight of the composition. The composition of the present invention does contain one or more fatty alcohol(s) having 18 or more carbon atoms. Said alcohol(s) is/are contained in a total amount higher than 0.05 % by weight in relation to the total weight of the composition.

Preferably, in the composition according to the present invention one or more fatty alcohol(s) having 16 or less carbon atoms is/are contained in a total amount of less than 5.5 % by weight, more preferably less than 5.0 % by weight, in relation to the total weight of the composition. The compositions of the present invention do contain one or more fatty alcohol(s) having 16 or less carbon atoms. Said alcohol(s) is/are contained in a total amount higher than 0.05 % by weight in relation to the total weight of the composition.

Silicones are synthetic components, mostly in form of polymers, containing silicium atoms connected via oxygen atoms. The functional unit mainly consisting of silicium atom and oxygen atom is called siloxane, has the general formula: $R_nSiO_{(4-n)/2}$ (n=0, 1, 2, 3).

In the meaning of the present invention the composition according to the present invention is free from silicone components. The term "free from" is intended to mean a composition containing less than 0.1 % by weight, preferably less than 0.01 % by weight, more preferably 0.001 % by weight, most preferably 0 % by weight, of a respective compound, relative to the total weight of the composition.

In addition to the above mentioned emollient one or more moisturizer(s) may be contained in the composition according to the invention.

Advantageously said moisturizers contain hydrophilic residues, which may lead to substances being hygroscopic. Hygroscopic substances bind water and therefore may provide moisture. The hygroscopic quality is due to hydrophilic substituents of the molecule, in many cases hydroxyl groups, but other functional groups, such as amine or carboxyl groups can fulfill the same function. Examples of moisturizers are propylene glycol, hexylene glycol, and butylene glycol, glyceryl triacetate, glycerol, sorbitol, xylitol, maltitol, poly dextrose, hydrogenated starch hydrolysate, urea, Aloe vera gel, alpha hydroxy acids such as lactic acid, honey, and/or polyethylene glycols such as PEG-4, PEG- 6, PEG-7, PEG-8, PEG-9, PEG-10, PEG-12, PEG-14, PEG-16, PEG-18, PEG-20, PEG-32, PEG-40, PEG-55, PEG-60, PEG-75, PEG-90, PEG-100, PEG-135, PEG-150, PEG-154, PEG-180, PEG-200, PEG-240. It is preferred, if at least one moisturizer having a smaller molecular weight, especially a molecular weight below 250 g/mol and at least one moisturizer having a higher molecular weight, especially a molecular weight above 500 g/mol, further especially above 1000 g/mol in the composition of the present invention. It is more preferred, if glycerine and PEG-150 are contained in the composition of the present invention. It is most preferred, if in relation to moisturizer only glycerine and PEG-150 are contained in the composition of the present invention.

In the composition of the present invention one or more moisturizer is/are contained in a total amount of 0.05 to 17 % by weight, preferably 0.1 to 12 % by weight, in relation to the total weight of the composition.

Additionally, at least one cellulose compound may be contained in the composition of the present invention. Cellulose compounds also comprise cellulose derived cellulose compounds, such as cellulose mixed ethers. Cellulose compounds are characterized by a chemical structure containing links of glucose residues, which are linked via β-1,4-bonds. In addition to unsubstituted celluloses, cellulose derivatives may be anionic, cationic, amphoteric or nonionic. Cellulose ethers, cellulose esters and cellulose ether esters have to be distinguished from these derivatives. Examples for cellulose esters are inorganic cellulose esters (nitrates, sulphates or phosphates of cellulose) and organic cellulose esters (monoacetates, triacetates, amidopropionates, acetate butyrates, acetate propionates or acetate trimellitates of cellulose) and mixed organic/inorganic esters of cellulose, such as acetate butyrate sulphates and acetate propionate sulphates of cellulose. Of the cellulose ether esters, the phthalates of hydroxypropylmethylcellulose and the sulphates of ethylcellulose may be mentioned.

According to the present invention cellulose compounds are selected from the non-ionic cellulose ethers. Nonionic cellulose ethers include alkyl celluloses, such as methyl celluloses and ethyl celluloses; hydroxyalkyl celluloses, such as hydroxymethyl celluloses, hydroxyethyl celluloses and hydroxypropyl celluloses; mixed hydroxyalkyl alkyl celluloses, also known as mixed cellulose ethers.

According to the present invention it is preferred, if at least one hydroxyethyl cellulose is contained in the composition according to the present invention. A suitable hydroxyethyl cellulose may be obtained from Ashland under the trade name Natrosol™ 250 HHR or from Dow under the trade name CELLOSIZE™ PCG-10, for example.

The at least one cellulose compound, especially the at least one hydroxyethyl cellulose is contained in a total amount of 0.05 % by weight to 3.0 % by weight, preferably from 0.1 % by weight to 2.0 % by weight, in relation to the total weight of the composition and in relation to the active content.

According to the invention, at least one cationic surfactant may be contained in the composition of the present invention. Cationic surfactants are preferably those, which have conditioning qualities. Moreover, said cationic surfactants may have emulsifying properties. More preferably, suitable cationic surfactants are chosen from quaternary ammonium salts. It is still more preferred that the quaternary ammonium salts contain nitrogen atoms having three methyl groups as substituents, whereas the fourth substituent is a long-chained alkyl residue which may be straight, branched, substituted or non-substituted. It is preferred, if the alkyl residue is a straight, non-substituted hydrocarbon group containing 12 to 28 carbon atoms. It is even more preferred if the alkyl residue contains 18 to 24 carbon atoms. The corresponding anion may be selected from the group of chloride, bromide and methosulfate. It is most preferred to use behentrimonium chloride, which may be purchased as Genamin KDMP from Clariant Produkte GmbH (Germany).

If at least one cationic surfactant is contained in the composition of the present invention, the at least one cationic surfactant is contained in an amount of 0.1 to 3.0 % by weight, more preferably 0.2 to 2.5 % by weight, in relation to the total weight of the composition. The values are referring to the active content of the cationic surfactants.

Additionally, the composition according to the present invention may comprise at least one preservative. Suitable preservatives are those ones, which are allowed and suitable for cosmetic compositions. However, it is preferred to use of Benzyl Alcohol, Methylisothiazolinone, Methylchloroisothiazolinone, Ethylparaben, Methylparaben, Phenoxyethanol, and mixtures thereof.

The preferred preservatives may be used alone or in combination. If at least one preservative is comprised in the composition of the present invention, the at least one preservative is contained in a total amount of 0.1 to 1.0 % by weight, preferably 0.4 to 0.9 % by weight, in relation to the total weight of the composition. The values are referring to the active content of the preservatives.

The composition according to the invention preferably has a pH value of 3.0 to 6.9, more preferably 3.1 to 6.5. The pH value may be adjusted by every physiologically suitable means, however it is preferred, if organic acids are used to adjust the pH value, more preferably citric acid.

The composition according to the invention preferably has a viscosity of 2,000 to 15,000 mPa·s, more preferably 2,100 to 12,000 mPa·s. The viscosity is determined by using a Rheomat R123 at 25°C.

The composition of the present invention may be prepared by any technique known or effective to prepare a hair conditioning composition. The process to prepare the composition of the present invention comprises conventional formulating and mixing techniques.

Specifically, compositions of the present invention and comparative compositions are preferably prepared using the following procedure:

For manufacturing a batch of one of the compositions mentioned in the present invention, an IKA RW20 digital or IKA Eurostar stir equipment and a stirrer, e.g. a paddle stirrer may be used.

- Heat oil phase, containing fatty alcohols, emollient(s), cellulose(s) and emulsifiers to 70-85°C, while stirring, stir until all components are dissolved.
- Heat water phase, containing water, moisturizer(s) and cationic surfactant(s) to 70-85°C, while stirring, stir until all components are dissolved.
- Mix oil phase and water phase and homogenize until a uniform composition is achieved.
- Cool the mixture to 45°C while stirring, add fragrance, preservative and optional further ingredients.
- Keep stirring until a uniform composition is achieved.

Products according to the examples 1, 2 and 4 (table 1) were prepared. For the measurement of the wet and dry combing force as well as the measurement of suppleness equal amounts of each product were applied to 7 tresses (each 2g, European hair, damaged/bleached) per product. The tresses were prewashed using a standard shampoo (having only cleansing and no caring properties), the water having a water hardness of 1.5 to 2.5 mmol/l $CaCO_3$. In relation to the measurement of dry combing force and the measurement of suppleness the tresses were dried under constant conditions for at least 18 hours, namely in a climatized room having 22°C $\pm$ 1°C and 55 % $\pm$ 5 % relative humidity.

The first measurements of wet and dry combing force as well as suppleness were performed generating the basic $t_0$ values. After application of equal amounts (0.2 g/g hair) of each conditioner product on the wet hair and rinsing off (90 seconds) the respective products the second measurement in relation to wet combing force was conducted generating the values $t_1$. For determining the respective values for dry combing force and suppleness the step of drying under constant conditions was included before the measurement was performed.

Analyzing the data of table 2 it becomes apparent that there are no significant differences between the products analyzed. All products have a conditioning effect in comparison to untreated hair. The differences seen between the products are only minor ones. Hence all products do have a comparable conditioning effect showing that the compositions according to the invention are as good in conditioning as comparative compositions, representing prior art compositions containing a silicone compound.

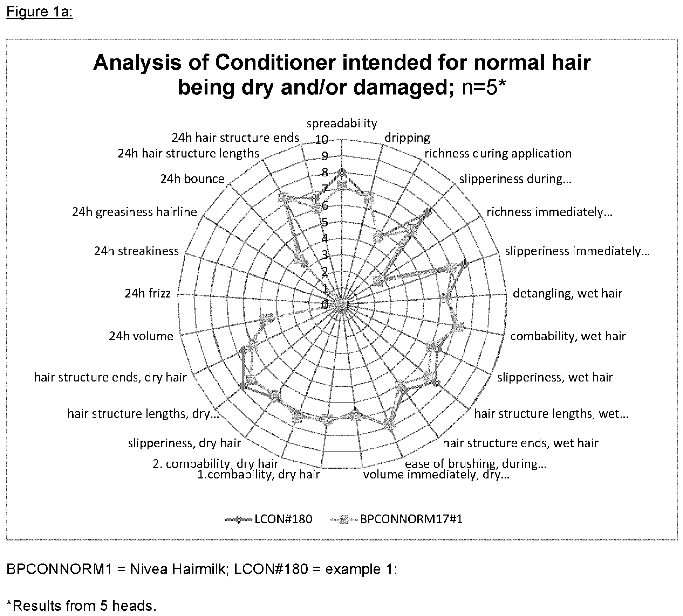

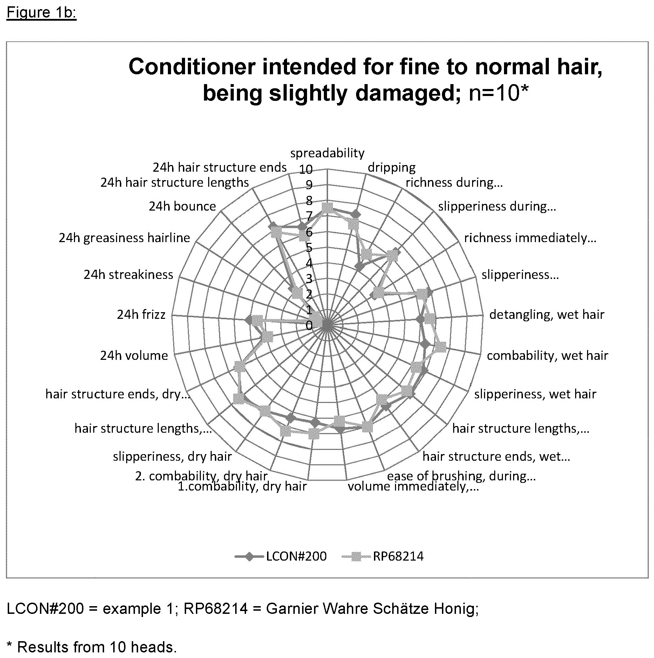

A further evaluation was conducted using a hair salon half head test. The hair of each test person was divided in two equal halves so that two different products can be compared directly. The hair was washed with a standard shampoo and then the two conditioner products to be compared were applied, each one on one side of the hair. Trained experts treated and evaluated the hair referring to a set of properties. The data are shown in figures 1a and 1b.

In a first test a market product (Nivea Hairmilk, representing the comparative product) was compared to an inventive product according to example 1 (figure 1a). The INCI listing of Nivea Hairmilk reads as follows: Aqua, Cetyl Alcohol, Myristyl Alcohol, Dimethicone, Stear-amidopropyl Dimethylamine, Lanolin Alcohol (Eucerit), Panthenol, Hydrolyzed Milk Protein, Silicone Quaternium-18, Guar Hydroxypropyltrimonium Chloride, Cocamidopropyl Betaine, Coco-Betaine, Trideceth-6, Sodium Chloride, Trideceth-12, C12-15 Pareth-3, Lactic Acid, Phenoxyethanol, Limonene, Linalool, Geraniol, Benzyl Alcohol, Citronellol, Alpha-Isomethyl Ionone, Parfum.

It becomes apparent that in some properties Nivea Hairmilk (comparative product) was evaluated better than the inventive product and vice versa. Summing up the evaluations, the inventive product is evaluated slightly better than the comparative product.

In a second test the inventive product according to example 1 was compared to another market product, namely Garnier Wahre Schätze Honig (representing the comparative product) (figure 1b). The INCI listing of Garnier Wahre Schätze Honig reads as follows: Aqua / Water, Cetyl Alcohol, PEG-180, CI 19140 / Yellow 5, CI 15985 / Yellow 6, Elaeis Guineensis Oil / Palm Oil, Hydroxyethylcellulose, Dodecene, Mel / Honey, Chlorhexidine Digluconate, Polox-amer 407, Limonene, Benzyl Alcohol, Benzyl Salicylate, Isopropyl Myristate, 2-Oleamido-1,3-Oczadecanediol, Propolis Extract, Myristyl Alcohol, Cetrimonium Chloride, Cetyl Esters, BHT, Citric Acid, Lauryl PEG/PPG-18/18 Methicone, Coumarin, Royal Jelly, Parfum / Fragrance.

As seen in the second test the comparative product was evaluated in some properties better than the inventive product and vice versa. Overall however, the inventive product was evaluated slightly better than the comparative product.

Looking at the measurement of wet and dry combing force as well as suppleness and the results of the hair salon half head tests it is obvious that the inventive compositions result at least in comparable properties of the hair in relation to the comparative compositions, and in the hair salon half head tests even have better results than the comparative products.

To verify the light texture of the composition an indirect means was used. Compositions having a rich, compact texture often weigh down hair resulting in less volume of the hair. Furthermore, after application of a composition with a rich, compact texture experts can feel residues left on the hair. In the past, many conditioners were developed with the aim to effectively deposit benefit ingredients on the hair. In consequence, touching hair treated in such a way an impression of residues left on the hair was perceived.

A comparison of inventive compositions (table 3, examples 5 and 7) to a comparative composition, exemplified by a silicone-containing composition (table 3, example 6), was conducted using a panel test, which conducted as described:

Caucasian bleached hair tresses were used. The evaluation of the hair tresses was conducted by trained panelists:

- Wet the hair with water;
- Fill a syringe with 2 ml of a composition according to the invention or a market product, evenly apply on the hair tress, and evaluate

  ◦ richness,
  ◦ ability of gliding and

- Wash off the product (one hand is used);
- Evaluate

  ◦ gliding,
  ◦ rinse ability and
  • Press out water softly;

- Evaluate

  ◦ combing,
  ◦ gliding and

- When hair is dry;
- Finally evaluate

  ◦ Volume

○ Residue.

The evaluation was based on a score of 10 points, "0 point" corresponds to "bad", and "10 points" corresponds to "very good".

| ***more volume | ** middle volume | * less volume |
| ***more residue | ** middle residue | * less residue. |

It becomes apparent (examples, table 3, lower part) that after the application of the compositions according to the invention more volume is provided to the hair. Furthermore, after using the inventive compositions the perception of residues left on the hair is significantly reduced.

## Brief description of the drawings

Fig.1a shows differences of results from 5 heads between example 1 of the present invention and control (Nivea Hairmilk); and
Fig.1b showsdifferences of results from 10 heads between example 1 of the present invention and control (Garnier Wahre Schätze Honig).

## Examples

The examples below are intended to illustrate the present invention without limiting it. The numerical values in the examples are percentages by weight, based on the total weight of the particular compositions, given as active content.

Table 1: Examples 1-4

| All the values given are referring to active content, if not denoted otherwise. | | | | |
|---|---|---|---|---|
| **INCI** | **1** | **2** | **3** | **4*** |
| | m [g] | m [g] | m [g] | m [g] |
| Aqua | Ad 100 | Ad 100 | Ad 100 | Ad 100 |
| Citric Acid | 0.01 | 0.01 | 0.01 | 0.01 |
| Cetyl Alcohol | 4.0 | 4.0 | 4.0 | 4.0 |
| Isopropyl Myristate | 0.5 | 0.5 | | 0.5 |
| Phenoxyethanol | 0.5 | 0.5 | 0.5 | 0.5 |
| Glycerin | 2 | 2 | 2 | 2 |
| Hydroxyethylcellulose | 1.0 | 1.0 | 1.0 | 1.0 |
| PEG-12 Dimethicone | | | | 0.5 |
| Stearyl Alcohol | 1.0 | 1.0 | 1.0 | 1.0 |
| Polyglyceryl-3 Methylglucose Distearate | 0.2 | 0.2 | 0.2 | 0.2 |
| Behentrimonium Chloride | 1.4 | 1.4 | 1.4 | 1.4 |
| Perfume | 0.7 | 0.7 | 0.7 | 0.7 |
| Isoamyl Laurate | 0.5 | | 0.5 | |
| * comparative example | | | | |

Table 2: Measurement of wet and dry combing force as well as suppleness:

| | | 1 | | | 2 | | | 4 | | |
|---|---|---|---|---|---|---|---|---|---|---|
| | | $t_0$ | $t_1$ | | $t_0$ | $t_1$ | | $t_0$ | $t_1$ | |
| Wet Combing Force [mN] | mean value | 592.49 | 121.08 | | 598.14 | 118.89 | | 558.81 | 116.51 | |
| | standard deviation | 48.01 | 7.53 | | 30.5 | 4.59 | | 55.15 | 4.12 | |
| | relative changes to $t_0$* | | | -79.56% | | | -80.12% | | | -80.21% |
| | | | | | | | | | | |
| Suppleness measurement [mN] | mean value | 192.92 | 146.6 | | 192.02 | 145.96 | | 192.76 | 151.56 | |
| | standard deviation | 5.87 | 4.75 | | 9.34 | 3 | | 8.94 | 3.12 | |
| | relative changes to $t_0$* | | | -24.01% | | | -23.99% | | | -21.37% |
| | | | | | | | | | | |
| Dry Combing Force [mN] | mean value | 10.81 | 7.44 | | 10.51 | 6.42 | | 10.73 | 6.86 | |
| | standard deviation | 1.01 | 0.8 | | 1.01 | 0.48 | | 1.51 | 0.63 | |
| | relative changes to $t_0$* | | | -31.08% | | | -38.92% | | | -36.07% |
| * [$(t_1-t_0)/t_0$] | | | | | | | | | | |

Table 3: Further examples 5-7 and results of a panel test

| All the values given are referring to active content, if not denoted otherwise. | | | |
|---|---|---|---|
| INCI | 5 | 6* | 7 |
| | m [g] | m [g] | m [g] |
| Aqua | Ad 100 | Ad 100 | Ad 100 |
| Myristyl Alcohol | 1 | 1 | 1 |
| Cetyl Alcohol | 3.5 | 3.5 | 3.5 |
| Isopropyl Myristate | 0.5 | | 0.5 |
| Phenoxyethanol | 0.5 | 0.5 | 0.5 |
| Glycerin | 5 | 5 | 5 |
| Hydroxyethylcellulose | 0.3 | 0.3 | 0.3 |
| Stearyl Alcohol | 1 | 1 | 1 |
| Behentrimonium Chloride | 1.8 | 1.8 | 1.8 |
| Perfume | 0.7 | 0.7 | 0.7 |
| Dimethicone | | 0.5 | |
| Isoamyl Laurate | | | 0.5 |

(continued)

| * comparative | | | |
|---|---|---|---|
| **HAIR PANEL DATA** | **5** | **6\*** | **7** |
| Gliding after 30s (during applicaiton) | 6 | 7 | 7 |
| Gliding immediately (rinsing) | 6 | 6 | 6 |
| Gliding (wet hair) | 5 | 6 | 6 |
| Combing (wet) | 8 | 9 | 9 |
| Volume (dry hair) | \*\*\* | \*\* | \*\*\* |
| residue (wet hair) | \* | \*\*\* | \*\* |
| residue (dry hair) | \* | \*\*\* | \* |
| \*\*\*more volume \* less volume \*\* middle volume | | | |
| \*\*\*more residue \* less residue \*\* middle residue | | | |

Table 4: Further examples8-11

| All the values given are referring to active content, if not denoted otherwise. | | | | |
|---|---|---|---|---|
| | **8** | **9** | **10** | **11** |
| **INCI** | m [g] | m [g] | m [g] | m [g] |
| Aqua | Ad 100 | Ad 100 | Ad 100 | Ad 100 |
| Cetyl Alcohol | 4.0 | 4.0 | 4.0 | 4.0 |
| Stearyl Alcohol | 1.5 | 1.5 | 1.5 | 1.5 |
| Hydroxyethylcellulose | 0.2 | 0.2 | 0.2 | 0.2 |
| PEG-150 | 0.5 | 0.5 | 0.5 | 0.5 |
| Isoamyl Laurate | 0.5 | 0.5 | | |
| Isopropyl Myristate | 0.5 | | 0.5 | 0.5 |
| Phenoxyethanol | 0.5 | 0.5 | 0.5 | 0.5 |
| Citric Acid | 0.01 | 0.01 | 0.01 | 0.01 |
| Glycerin | 2 | 2 | 2 | 2 |
| PEG-12 Dimethicone | | | | 0.5 |
| Polyglyceryl-3 Methylglucose Distearate | 0.2 | 0.2 | 0.2 | 0.2 |
| Behentrimonium Chloride | 2 | 2 | 2 | 2 |
| Perfume | 0.7 | 0.7 | 0.7 | 0.7 |

**Claims**

1. A conditioning composition, containing

    - at least one emollient chosen from ester oils, semi synthetic triglycerides, alkanes having from 8 to 20 carbon atoms, and/or poly-C2-C16-olefins, especially hydrogenated poly-C2-C16-olefins,
    - at least one emulsifier, preferably at least one O/W emulsifier, and/or at least one cationic surfactant,
    - a combination of fatty alcohols, wherein one or more fatty alcohol(s) having 18 or more carbon atoms is/are contained in a total amount of less than 2.0 % by weight and one or more fatty alcohol(s) having 16 or less carbon atoms is/are contained in a total amount of less than 6.0 % by weight, respectively each one in relation to the total weight of the composition,
    wherein the composition is free from silicones,

wherein the at least one emulsifier is contained in a total amount of 0.05 to 3.0 % by weight, in relation to the total weight of the composition, and

wherein the at least one O/W emulsifier is chosen from glyceryl stearate citrate, glyceryl stearate SE, polyglyceryl-3 distearate in combination with glyceryl stearate citrate, and/or polyglyceryl-3 methyl glucose distearate, and

wherein water is contained in an amount of at least 75 % by weight, preferably 85 to 98 % by weight water, more preferably 88 to 95 % by weight water, in relation to the total weight of the composition .

2. The composition according to claim 1 **characterized in that** the composition is an emulsion, preferably an O/W emulsion.

3. The composition according to claim 1 or 2 **characterized in that** the at least one emulsifier is polyglyceryl-3 methyl glucose distearate.

4. The composition according to any of the preceding claims **characterized in that** the at least one emulsifier is contained in a total amount of 0.1 to 2.0 % by weight, in relation to the total weight of the composition.

5. The composition according to any of the preceding claims **characterized in that** the at least one cationic surfactant is contained, preferably at least one quaternary ammonium salts, more preferably behentrimonium chloride.

6. The composition according to any of the preceding claims **characterized in that** the at least one cationic surfactant is contained in a total amount of 0.1 to 3.0 % by weight, preferably 0.2 to 2.5 % by weight, in relation to the total weight of the composition.

7. The composition according to any of the preceding claims **characterized in that** the at least one emollient is chosen from at least one ester oil, preferably octyl palmitate, octyl cocoate, octyl isostearate, octyldodecyl myristate, cetearyl isononanoate, isopropyl myristate, isopropyl palmitate, isopropyl stearate, isopropyl oleate, n-butyl stearate, n-hexyl laurate, n-decyl oleate, isooctyl stearate, isononyl stearate, isononyl isononanoate, 2-ethylhexyl palmitate, 2-ethylhexyl laurate, 2-ethylhexyl stearate, 2-hexyldecyl stearate, 2-octyldodecyl palmitate, stearyl heptanoate, oleyl oleate, oleyl erucate, erucyl oleate, erucyl erucate, tridecyl stearate, tridecyl trimellitate, isoamyl laurate, more preferably from isopropyl myristate and/or isoamyl laurate.

8. The composition according to claim 7 **characterized in that** the at least one ester oil is contained in a total amount of 0.1 to 5.0% by weight, preferably 0.4 to 3.0 % by weight, in relation to the total weight of the composition.

9. The composition according to any of the preceding claims **characterized in that** the at least one emollient is chosen from at least one poly-C2-C16-olefin, preferably from polybutene, polyisobutene and polydecene, more preferably from hydrogenated polybutene, polyisobutene and Polydecene, most preferably from hydrogenated polyisobutene and/or hydrogenated polydecene.

10. The composition according to claim 9 **characterized in that** the at least one poly-C2-C16-olefin is contained in a total amount of 0.05 to 6.0% by weight, preferably 0.1 to 4.0 % by weight, relative to the total weight of the composition.

11. The composition according to any of the preceding claims **characterized in that** the at least one emollient is chosen from at least one semi synthetic triglyceride, preferably caprylic/capric triglyceride.

12. The composition according to claim 11 **characterized in that** the at least one semi synthetic triglyceride is contained in a total amount of 0.05 to 6.0 % by weight, preferably 0.1 to 4.0 % by weight, relative to the total weight of the composition.

13. The composition according to any of the preceding claims **characterized in that** the at least one emollient is chosen at least one alkane having from 8 to 20 carbon atoms, especially mixtures thereof, preferably undecane/tridecane.

14. The composition according to claim 13 **characterized in that** the at least one alkane having from 8 to 20 carbon atoms is contained in a total amount of 0.05 to 6.0 % by weight, preferably 0.1 to 4.0 % by weight, relative to the total weight of the composition.

15. The composition according to any of the preceding claims **characterized in that** one of the fatty alcohol(s) having 18 or more carbon atoms is stearyl alcohol, preferably the fatty alcohol having 18 or more carbon atoms is stearyl alcohol.

16. The Composition according to any of the preceding claims **characterized in that** myristyl alcohol and/or cetyl alcohol is/are part of the group of fatty alcohols having 16 or less carbon atoms, preferably the fatty alcohols having 16 or less carbon atoms are myristyl alcohol and cetyl alcohol.

17. The composition according to any of the preceding claims **characterized in that** the at least one of the fatty alcohol(s) having 18 or more carbon atoms is contained in a total amount of less than 1.8 % by weight, preferably less than 1.5 % by weight, in relation to the total weight of the composition.

18. The composition according to any of the preceding claims **characterized in that** the at least one of the fatty alcohol(s) having 18 or more carbon atoms is contained in a total amount of higher than 0.05 % by weight, in relation to the total weight of the composition.

19. The composition according to any of the preceding claims **characterized in that** the at least one of the fatty alcohol(s) having 16 or less carbon atoms is contained in a total amount of less than 5.5 % by weight, preferably less than 5.0 % by weight, in relation to the total weight of the composition.

20. The composition according to any of the preceding claims **characterized in that** the at least one of the fatty alcohol(s) having 16 or less carbon atoms is contained in a total amount of higher than 0.05 % by weight, in relation to the total weight of the composition.

21. The composition according to any of the preceding claims **characterized in that** additionally one or more moisturizer(s) are contained, preferably chosen from propylene glycol, hexylene glycol, and butylene glycol, glyceryl triacetate, glycerol, sorbitol, xylitol, maltitol, poly dextrose, hydrogenated starch hydrolysate, urea, Aloe vera gel, alpha hydroxy acids such as lactic acid, honey, and/or polyethylene glycols such as PEG-4, PEG-6, PEG-7, PEG-8, PEG-9, PEG-10, PEG-12, PEG-14, PEG-16, PEG-18, PEG-20, PEG-32, PEG-40, PEG-55, PEG-60, PEG-75, PEG-90, PEG-100, PEG-135, PEG-150, PEG-154, PEG-180, PEG-200, PEG-240, more preferably chosen from glycerine and PEG-150.

22. The composition according to claim 21 **characterized in that** the at least one moisturizer is contained in a total amount of 0.05 to 17 % by weight, preferably 0.1 to 12 % by weight, in relation to the total weight of the composition.

23. The composition according to any of the preceding claims **characterized in that** additionally at least one cellulose compound is contained, preferably at least one hydroxyalkyl cellulose, more preferably at least one hydroxyethyl cellulose.

24. The composition according to claim 23 **characterized in that** the at least one cellulose compound is contained in a total amount of 0.05 to 3.0 % by weight, preferably 0.1 to 2.0 % by weight, in relation to the total weight of the composition.

**Patentansprüche**

1. Konditionierende Zusammensetzung, enthaltend

- mindestens ein Emolliens, ausgewählt aus Esterölen, halbsynthetischen Triglyceriden, Alkanen mit 8 bis 20 Kohlenstoffatomen und/oder Poly-C2-C16-olefinen, insbesondere hydrierten oder Poly-C2-C16-olefinen,
- mindestens einen Emulgator, vorzugsweise mindestens einen O/W-Emulgator, und/oder mindestens ein kationisches Tensid,
- eine Kombination von Fettalkoholen, wobei ein Fettalkohol oder mehrere Fettalkohole mit 18 oder mehr Kohlenstoffatomen in einer Gesamtmenge von weniger als 2,0 Gew.-% enthalten ist/sind und ein Fettalkohol oder mehrere Fettalkohole mit 16 oder weniger Kohlenstoffatomen in einer Gesamtmenge von weniger als 6,0 Gew.-% enthalten ist/sind, jeweils bezogen auf das Gesamtgewicht der Zusammensetzung,
wobei die Zusammensetzung frei von Silikonen ist,
wobei mindestens ein Emulgator in einer Gesamtmenge von 0,05 bis 3,0 Gew.-%, bezogen auf das Gesamtgewicht der Zusammensetzung, enthalten ist und
wobei der mindestens eine O/W-Emulgator aus Glycerylstearatcitrat, Glycerylstearat SE, Polyglyceryl-3-distearat in Kombination mit Glycerylstearatcitrat und/oder Polyglyceryl-3-methylglucosedistearat ausgewählt ist und

wobei Wasser in einer Menge von mindestens 75 Gew.-%, vorzugsweise 85 bis 98 Gew.-% Wasser, weiter bevorzugt 88 bis 95 Gew.-% Wasser, bezogen auf das Gesamtgewicht der Zusammensetzung, enthalten ist.

2. Zusammensetzung nach Anspruch 1, **dadurch gekennzeichnet, dass** es sich bei der Zusammensetzung um eine Emulsion, vorzugsweise eine O/W-Emulsion, handelt.

3. Zusammensetzung nach Anspruch 1 oder 2, **dadurch gekennzeichnet, dass** es sich bei dem mindestens einen Emulgator um Polyglyceryl-3-methylglucosedistearat handelt.

4. Zusammensetzung nach einem der vorhergehenden Ansprüche, **dadurch gekennzeichnet, dass** der mindestens eine Emulgator in einer Menge von 0,1 bis 2,0 Gew.-%, bezogen auf das Gesamtgewicht der Zusammensetzung, enthalten ist.

5. Zusammensetzung nach einem der vorhergehenden Ansprüche, **dadurch gekennzeichnet, dass** das mindestens eine kationische Tensid, vorzugsweise mindestens ein quartäres Ammoniumsalz, weiter bevorzugt Behentrimoniumchlorid, enthalten ist.

6. Zusammensetzung nach einem der vorhergehenden Ansprüche, **dadurch gekennzeichnet, dass** das mindestens eine kationische Tensid in einer Gesamtmenge von 0,1 bis 3 Gew.-%, vorzugsweise 0,2 bis 2,5 Gew.-%, bezogen auf das Gesamtgewicht der Zusammensetzung, enthalten ist.

7. Zusammensetzung nach einem der vorhergehenden Ansprüche, **dadurch gekennzeichnet, dass** das mindestens eine Emolliens aus mindestens einem Esteröl, vorzugsweise Octylpalmitat, Octylcocoat, Octylisostearat, Octyldodecylmyristat, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Ethylhexylstearat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, Isoamyllaurat, weiter bevorzugt aus Isopropylmyristat und/oder Isoamyllaurat, ausgewählt ist.

8. Zusammensetzung nach Anspruch 7, **dadurch gekennzeichnet, dass** das mindestens eine Esteröl in einer Gesamtmenge von 0,1 bis 5,0 Gew.-%, vorzugsweise 0,4 bis 3,0 Gew.-%, bezogen auf das Gesamtgewicht der Zusammensetzung, enthalten ist.

9. Zusammensetzung nach einem der vorhergehenden Ansprüche, **dadurch gekennzeichnet, dass** das mindestens eine Emolliens aus mindestens einem Poly-C2-C16-olefin, vorzugsweise aus Polybuten, Polyisobuten und Polydecen, weiter bevorzugt aus hydriertem Polybuten, Polyisobuten und Polydecen, ganz besonders bevorzugt aus hydriertem Polyisobuten und hydriertem Polydecen, ausgewählt ist.

10. Zusammensetzung nach Anspruch 9, **dadurch gekennzeichnet, dass** das mindestens eine Poly-C2-C16-olefin in einer Gesamtmenge von 0,05 bis 6,0 Gew.-%, vorzugsweise 0,1 bis 4,0 Gew.-%, bezogen auf das Gesamtgewicht der Zusammensetzung, enthalten ist.

11. Zusammensetzung nach einem der vorhergehenden Ansprüche, **dadurch gekennzeichnet, dass** das mindestens eine Emolliens aus mindestens einem halbsynthetischen Triglycerid, vorzugsweise Capryl-/Caprinsäuretriglycerid, ausgewählt ist.

12. Zusammensetzung nach Anspruch 11, **dadurch gekennzeichnet, dass** das mindestens eine halbsynthetische Triglycerid in einer Gesamtmenge von 0,05 bis 6,0 Gew.-%, vorzugsweise 0,1 bis 4,0 Gew.-%, bezogen auf das Gesamtgewicht der Zusammensetzung, enthalten ist.

13. Zusammensetzung nach einem der vorhergehenden Ansprüche, **dadurch gekennzeichnet, dass** das mindestens eine Emolliens aus mindestens einem Alkan mit 8 bis 20 Kohlenstoffatomen, insbesondere Mischungen davon, vorzugsweise Undecan/Tridecan, ausgewählt ist.

14. Zusammensetzung nach Anspruch 13, **dadurch gekennzeichnet, dass** das mindestens eine Alkan mit 8 bis 20 Kohlenstoffatomen in einer Gesamtmenge von 0,05 bis 6,0 Gew.-%, vorzugsweise 0,1 bis 4,0 Gew.-%, bezogen auf das Gesamtgewicht der Zusammensetzung, enthalten ist.

**15.** Zusammensetzung nach einem der vorhergehenden Ansprüche, **dadurch gekennzeichnet, dass** es sich bei einem der Fettalkohole mit 18 oder mehr Kohlenstoffatomen um Stearylalkohol handelt und es sich vorzugsweise bei dem Fettalkohol mit 18 oder mehr Kohlenstoffatomen um Stearylalkohol handelt.

**16.** Zusammensetzung nach einem der vorhergehenden Ansprüche, **dadurch gekennzeichnet, dass** Myristylalkohol und/oder Cetylalkohol Teil der Gruppe von Fettalkoholen mit 16 oder weniger Kohlenstoffatomen ist/sind und es sich vorzugsweise bei dem Fettalkohol mit 16 oder weniger Kohlenstoffatomen um Myristylalkohol und Cetylalkohol handelt.

**17.** Zusammensetzung nach einem der vorhergehenden Ansprüche, **dadurch gekennzeichnet, dass** der mindestens eine der Fettalkohole mit 18 oder mehr Kohlenstoffatomen in einer Gesamtmenge von weniger als 1,8 Gew.-%, vorzugsweise weniger als 1,5 Gew.-%, bezogen auf das Gesamtgewicht der Zusammensetzung, enthalten ist.

**18.** Zusammensetzung nach einem der vorhergehenden Ansprüche, **dadurch gekennzeichnet, dass** der mindestens eine der Fettalkohole mit 18 oder mehr Kohlenstoffatomen in einer Gesamtmenge von mehr als 0,05 Gew.-%, bezogen auf das Gesamtgewicht der Zusammensetzung, enthalten ist.

**19.** Zusammensetzung nach einem der vorhergehenden Ansprüche, **dadurch gekennzeichnet, dass** der mindestens eine der Fettalkoholen mit 16 oder weniger Kohlenstoffatomen in einer Gesamtmenge von weniger als 5,5 Gew.-%, vorzugsweise weniger als 5,0 Gew.-%, bezogen auf das Gesamtgewicht der Zusammensetzung, enthalten ist.

**20.** Zusammensetzung nach einem der vorhergehenden Ansprüche, **dadurch gekennzeichnet, dass** der mindestens eine der Fettalkohole mit 16 oder weniger Kohlenstoffatomen in einer Gesamtmenge von mehr als 0,05 Gew.-%, bezogen auf das Gesamtgewicht der Zusammensetzung, enthalten ist.

**21.** Zusammensetzung nach einem der vorhergehenden Ansprüche, **dadurch gekennzeichnet, dass** zusätzlich ein oder mehrere Feuchthaltemittel enthalten sind, die vorzugsweise aus Propylenglykol, Hexylenglykol und Butylenglykol, Glyceryltriacetat, Glycerin, Sorbitol, Xylitol, Maltitol, Polydextrose, hydriertem Stärkehydrolysat, Harnstoff, Aloe-vera-Gel, alpha-Hydroxysäuren wie Milchsäure, Honig und/oder Polyethylenglykolen wie PEG-4, PEG- 6, PEG-7, PEG-8, PEG-9, PEG-10, PEG-12, PEG-14, PEG-16, PEG-18, PEG- 20, PEG-32, PEG-40, PEG-55, PEG-60, PEG-75, PEG-90, PEG-100, PEG-135, PEG-150, PEG-154, PEG-180, PEG-200, PEG-240 ausgewählt sind und weiter bevorzugt aus Glycerin und PEG-150 ausgewählt sind.

**22.** Zusammensetzung nach Anspruch 21, **dadurch gekennzeichnet, dass** das mindestens eine Feuchthaltemittel in einer Gesamtmenge von 0,05 bis 17 Gew.-%, vorzugsweise 0,1 bis 12 Gew.-%, bezogen auf das Gesamtgewicht der Zusammensetzung, enthalten ist.

**23.** Zusammensetzung nach einem der vorhergehenden Ansprüche, **dadurch gekennzeichnet, dass** zusätzlich mindestens eine Celluloseverbindung enthalten ist, vorzugsweise mindestens ein Hydroxyalkylcellulose, weiter bevorzugt mindestens eine Hydroxyethylcellulose.

**24.** Zusammensetzung nach Anspruch 23, **dadurch gekennzeichnet, dass** die mindestens eine Celluloseverbindung in einer Gesamtmenge von 0,05 bis 3,0 Gew.-%, vorzugsweise 0,1 bis 2,0 Gew.-%, bezogen auf das Gesamtgewicht der Zusammensetzung, enthalten ist.

**Revendications**

**1.** Composition de conditionnement, contenant

- au moins un émollient choisi parmi les huiles d'ester, les triglycérides semi-synthétiques, les alcanes ayant de 8 à 20 atomes de carbone, et/ou les poly-oléfines en C2-C16, notamment les poly-oléfines en C2-C16 hydrogénées,
- au moins un émulsifiant, de préférence au moins un émulsifiant H/E, et/ou au moins un tensioactif cationique,
- une combinaison d'alcools gras, dans laquelle un ou plusieurs alcools gras ayant 18 atomes de carbone ou plus sont contenus en une quantité totale inférieure à 2,0 % en poids et un ou plusieurs alcools gras ayant 16 atomes de carbone ou moins est/sont contenu(s) en une quantité totale inférieure à 6,0 % en poids, respectivement chacune par rapport au poids total de la composition,

dans laquelle la composition est exempte de silicones,

dans laquelle l'au moins un émulsifiant est contenu en une quantité totale de 0,05 à 3,0 % en poids, par rapport au poids total de la composition, et

dans laquelle l'au moins un émulsifiant H/E est choisi parmi le stéarate citrate de glycéryle, le stéarate de glycéryle SE, le distéarate de polyglycéryl-3 en combinaison avec le stéarate citrate de glycéryle et/ou le distéarate de polyglycéryl-3 méthyl glucose, et

dans laquelle de l'eau est contenue en une quantité d'au moins 75 % en poids, de préférence de 85 à 98 % en poids d'eau, plus préférablement de 88 à 95 % en poids d'eau, par rapport au poids total de la composition.

2. Composition selon la revendication 1, **caractérisée en ce que** la composition est une émulsion, de préférence une émulsion H/E.

3. Composition selon la revendication 1 ou 2, **caractérisée en ce que** l'au moins un émulsifiant est le distéarate de polyglycéryl-3 méthylglucose.

4. Composition selon l'une quelconque des revendications précédentes, **caractérisée en ce que** l'au moins un émulsifiant est contenu en une quantité totale de 0,1 à 2,0 % en poids, par rapport au poids total de la composition.

5. Composition selon l'une quelconque des revendications précédentes, **caractérisée en ce que** l'au moins un tensioactif cationique est contenu, de préférence au moins un sel d'ammonium quaternaire, plus préférablement le chlorure de béhentrimonium.

6. Composition selon l'une quelconque des revendications précédentes, **caractérisée en ce que** l'au moins un tensioactif cationique est contenu en une quantité totale de 0,1 à 3,0 % en poids, de préférence de 0,2 à 2,5 % en poids, par rapport au poids total de la composition.

7. Composition selon l'une quelconque des revendications précédentes, **caractérisée en ce que** l'au moins un émollient est choisi parmi au moins une huile d'ester, de préférence le palmitate d'octyle, le cocoate d'octyle, l'isostéarate d'octyle, le myristate d'octyldodécyle, l'isononanoate de cétéaryle, le myristate d'isopropyle, le palmitate d'isopropyle, le stéarate d'isopropyle, l'oléate d'isopropyle, le stéarate de n-butyle, le laurate de n-hexyle, l'oléate de n-décyle, le stéarate d'isooctyle, le stéarate d'isononyle, l'isononanoate d'isononyle, le palmitate de 2-éthylhexyle, le laurate de 2-éthylhexyle, le stéarate de 2-éthylhexyle, le stéarate de 2-hexyldécyle, le palmitate de 2-octyldodécyle, l'heptanoate de stéaryle, l'oléate d'oléyle, l'érucate d'oléyle, l'oléate d'érucyle, l'érucate d'érucyle, le stéarate de tridécyle, le trimellitate de tridécyle, le laurate d'isoamyle, plus préférablement parmi le myristate d'isopropyle et/ou le laurate d'isoamyle.

8. Composition selon la revendication 7, **caractérisée en ce que** l'au moins une huile d'ester est contenue en une quantité totale de 0,1 à 5,0 % en poids, de préférence de 0,4 à 3,0 % en poids, par rapport au poids total de la composition.

9. Composition selon l'une quelconque des revendications précédentes, **caractérisée en ce que** l'au moins un émollient est choisi parmi au moins une poly-oléfine en C2-C16, de préférence parmi un polybutène, un polyisobutène et un polydécène, plus préférablement parmi un polybutène hydrogéné, un polyisobutène et un polydécène, le plus préférablement parmi un polyisobutène hydrogéné et/ou un polydécène hydrogéné.

10. Composition selon la revendication 9, **caractérisée en ce que** l'au moins une poly-oléfine en C2-C16 est contenue en une quantité totale de 0,05 à 6,0 % en poids, de préférence de 0,1 à 4,0 % en poids, par rapport au poids total de la composition.

11. Composition selon l'une quelconque des revendications précédentes, **caractérisée en ce que** l'au moins un émollient est choisi parmi au moins un triglycéride semi-synthétique, de préférence un triglycéride caprylique/caprique.

12. Composition selon la revendication 11, **caractérisée en ce que** l'au moins un triglycéride semi-synthétique est contenu en une quantité totale de 0,05 à 6,0 % en poids, de préférence de 0,1 à 4,0 % en poids, par rapport au poids total de la composition.

**13.** Composition selon l'une quelconque des revendications précédentes, **caractérisée en ce que** l'au moins un émollient est choisi parmi au moins un alcane ayant de 8 à 20 atomes de carbone, notamment des mélanges de ceux-ci, de préférence l'undécane/tridécane.

**14.** Composition selon la revendication 13, **caractérisée en ce que** l'au moins un alcane ayant de 8 à 20 atomes de carbone est contenu en une quantité totale de 0,05 à 6,0 % en poids, de préférence de 0,1 à 4,0 % en poids, par rapport au poids total de la composition.

**15.** Composition selon l'une quelconque des revendications précédentes, **caractérisée en ce que** l'un du ou des alcools gras ayant 18 atomes de carbone ou plus est l'alcool stéarylique, de préférence l'alcool gras ayant 18 atomes de carbone ou plus est l'alcool stéarylique.

**16.** Composition selon l'une quelconque des revendications précédentes, **caractérisée en ce que** l'alcool myristylique et/ou l'alcool cétylique fait/font partie du groupe des alcools gras ayant 16 atomes de carbone ou moins, de préférence les alcools gras ayant 16 atomes de carbone ou moins sont l'alcool myristylique et l'alcool cétylique.

**17.** Composition selon l'une quelconque des revendications précédentes, **caractérisée en ce que** l'au moins un du ou des alcools gras ayant 18 atomes de carbone ou plus est contenu en une quantité totale inférieure à 1,8 % en poids, de préférence inférieure à 1,5 % en poids, par rapport au poids total de la composition.

**18.** Composition selon l'une quelconque des revendications précédentes, **caractérisée en ce que** l'au moins un du ou des alcools gras ayant 18 atomes de carbone ou plus est contenu en une quantité totale supérieure à 0,05 % en poids, par rapport au poids total de la composition.

**19.** Composition selon l'une quelconque des revendications précédentes, **caractérisée en ce que** l'au moins un du ou des alcools gras ayant 16 atomes de carbone ou moins est contenu en une quantité totale inférieure à 5,5 % en poids, de préférence inférieure à 5,0 % en poids, par rapport au poids total de la composition.

**20.** Composition selon l'une quelconque des revendications précédentes, **caractérisée en ce que** l'au moins un du ou des alcools gras ayant 16 atomes de carbone ou moins est contenu en une quantité totale supérieure à 0,05 % en poids, par rapport au poids total de la composition.

**21.** Composition selon l'une quelconque des revendications précédentes, **caractérisée en ce qu'**en outre un ou plusieurs hydratants sont contenus, de préférence choisis parmi le propylène glycol, l'hexylène glycol et le butylène glycol, le triacétate de glycéryle, le glycérol, le sorbitol, le xylitol, le maltitol, le polydextrose, l'hydrolysat d'amidon hydrogéné, l'urée, le gel d'aloe vera, les alpha-hydroxyacides tels que l'acide lactique, le miel et/ou les polyéthylène glycols tels que le PEG-4, le PEG-6, le PEG-7, le PEG-8, le PEG-9, le PEG-10, le PEG-12, le PEG-14, le PEG-16, le PEG-18, le PEG-20, le PEG-32, le PEG-40, le PEG-55, le PEG-60, le PEG-75, le PEG-90, le PEG-100, le PEG-135, le PEG-150, le PEG-154, le PEG-180, le PEG-200, le PEG-240, plus préférablement choisis parmi la glycérine et le PEG-150.

**22.** Composition selon la revendication 21, **caractérisée en ce que** l'au moins un hydratant est contenu en une quantité totale de 0,05 à 17 % en poids, de préférence de 0,1 à 12 % en poids, par rapport au poids total de la composition.

**23.** Composition selon l'une quelconque des revendications précédentes, **caractérisée en ce qu'**en outre au moins un composé cellulosique est contenu, de préférence au moins une hydroxyalkylcellulose, plus préférablement au moins une hydroxyéthylcellulose.

**24.** Composition selon la revendication 23, **caractérisée en ce que** l'au moins un composé cellulosique est contenu en une quantité totale de 0,05 à 3,0 % en poids, de préférence de 0,1 à 2,0 % en poids, par rapport au poids total de la composition.

Figure 1a:

**Analysis of Conditioner intended for normal hair being dry and/or damaged; n=5***

BPCONNORM1 = Nivea Hairmilk; LCON#180 = example 1;

*Results from 5 heads.

Figure 1b:

**Conditioner intended for fine to normal hair, being slightly damaged; n=10***

LCON#200 = example 1; RP68214 = Garnier Wahre Schätze Honig;

* Results from 10 heads.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017036134 A1
- WO 2018018487 A1
- WO 2018227515 A1
- WO 2018095152 A1
- WO 2016090150 A1
- WO 2015066777 A1
- KR 20090054113 A

**Non-patent literature cited in the description**

- Database WPI, AN 2009-K33099